# EUROPEAN PATENT APPLICATION

(11) **EP 3 903 763 A1**
(43) Date of publication of application: **03.11.2021**
(21) Application number: 18944946.5
(22) Date of filing: 28.12.2018
(51) Int. Cl.: A61K 8/11, A61K 8/81, A61Q 19/00, B01J 13/04

(54) **METHOD FOR MANUFACTURING OIL-DISPERSED DOUBLE CAPSULE AND OIL-DISPERSED DOUBLE CAPSULE MANUFACTURED THEREFROM**

(71) Applicant: KPT Ltd, Chungcheongbuk-do 28162 (KR)
(72) Inventor: LEE, Jae Uk, Daejeon 34067 (KR); KIM, Su Hyun, Icheon-si, Gyeonggi-do 17422 (KR); SONG, Eun Ho, Cheongju-si, Chungcheongbuk-do 28166 (KR)
(74) Representative: BCKIP
(86) International application number: PCT/KR2018/016888
(87) International publication number: WO 2020/138569

(57) **Abstract**

The present invention provides a method for manufacturing an oil-dispersed double capsule and an oil-dispersed double capsule manufactured therefrom, the method comprising the following steps: (a) preparing an aqueous phase comprising a thickener and a curing agent, and an oil phase comprising a base oil and an encapsulating pigment; (b) mixing the oil phase with the aqueous phase obtained in step (a) to produce an oil-dispersed double capsule base; and (c) dropping the oil-dispersed double capsule base obtained in step (b) onto a dropping medium to produce an oil-dispersed double-capsule. As the method for manufacturing an oil-dispersed double capsule of the present invention can stabilize poorly soluble active ingredients in the cosmetic manufacturing field and improve aesthetics through various color control in the capsule, the method can be used to commercialize a skin care product or a base makeup product and can be effectively used as a technology that can easily and efficiently manufacture the same in a mass production process.

## Description

### TECHNICAL FIELD

The invention relates to a method for manufacturing oil-dispersed double capsule, and more particularly, a method for manufacturing oil-dispersed double capsule configured to encapsulate one kind or more of oil into water-soluble carrier, and an oil-dispersed double capsule manufactured therefrom.

### BACKGROUND ART

In the recent cosmetic industry, various emulsion techniques are applied such as nano emulsion, multiple emulsion, liquid crystal emulsion, pickering emulsion, and so on as a means for appearance differentiation and enhancement in delivering effective ingredients in a product.

Giant size of capsules, most typically used in the current cosmetic industry, include those using carrageenan and agar such as the capsule compositions for cosmetics disclosed in Korean Patent Publication No. 10-0342357 or those using sodium alginate and alkali metallic salt disclosed in Korean Patent Publication No. 10-0814034. However, as these capsules have undesirable collapse under pressure, and materials comprised of capsules after the collapsing are comprised of water-soluble polymer, there is disadvantage in which absorbing is not easy to the skin. Alternatively, a research relating to application of nano structured particles that can enhance delivering efficiency of materials while being friendly to the human body by sealing effective materials into nano particles such as liposome or cubosome is actively conducted.

With respect to this, as a capsule containing composition dispersed in aqueous solution, a capsule containing composition for external use has been reported in which a film of an oil capsule is comprised of elements selected from a group consisting of higher alcohol and glycerin having 16 carbon atoms or more, fatty acid ester, and icosane diacid condensate, and an aqueous solution contains water-soluble polymer. However, content of constituent elements is so high that content of effective elements is relatively small, resulting in limited commercialization into functional cosmetics.

Meanwhile, described is a composition for cosmetics containing micro-capsules as effective element generated from collection of active elements selected from a group consisting of 7-dihydrocholesterol, retinol, and oil-soluble licorice extract and stirring of one kind or more of solid wax selected from a group consisting of candelilla wax, behenyl alcohol, batyl alcohol, gylceryl stearate and ceresin wax in a mixing state of aqueous phase and oil phase. However, the above technique has disadvantage in which fabrication process becomes complicated for encapsulation of various active elements because it can generate one kind of micro-capsules with respect to one kind of active elements. Accordingly, development of new technology that can secure one kind or more of oils for effective improvement of the above problem is requested.

### DISCLOSURE OF INVENTION

### TECHNICAL PROBLEM

Inventors of the present invention conducted a research relating to new capsule formulation that can secure one kind or more of oil by mixing oil phase and aqueous phase without emulsifying using surfactant and discovered that properly increasing viscosity of aqueous phase leads to formation of an oil-dispersed double capsule in which aqueous phase and oil phase are not emulsified. Further, additionally confirmed is that two kinds or more of specific oils can be secured within one double capsule.

Accordingly, the objective of the present invention is to provide a method for manufacturing an oil-dispersed double capsule.

### SOLUTION TO PROBLEM

According to an aspect of the invention, there is provided a method for manufacturing an oil-dispersed double capsule including (a) manufacturing aqueous phase containing a thickener and a curing agent and oil phase containing base oil and an encapsulating pigment; (b) manufacturing an oil-dispersed double capsule base by mixing the aqueous phase and the oil phase obtained from (a); and (c) manufacturing an oil-dispersed double capsule by dropping the oil-dispersed double capsule base obtained from (b) to a dropping medium.

According to an embodiment, one kind or more of oil may be dispersed in the oil-dispersed double capsule.

In the manufacturing method of the invention, the thickener of (a) may be one kind or more selected from a group consisting of acrylate/C10-30alkylacrylate crosspolymer, carbomer, agar, carrageenan, guar gum, xanthan gum, alginic acid, pectin, starch, magnesium silicate, and sodium polyacrylate starch. Further, the base oil may be one kind or more selected from a group consisting of sunflower seed oil, macadamia seed oil, jojoba oil, drumstick seed oil, olive oil, grape seed oil, coconut oil, almond oil, camellia oil, avocado oil, caprlic/caprictriglyceride, caprlic/capricglyceride, dimethicone, amodimethicone, penyltrimethicone and dimethicone/vinyldimethicone crosspolymer, and the base oil may be included in 0.1 - 50 wt.%.

In the manufacturing method of the invention, the oil-dispersed double capsule base of (c) may be kept at the temperature of 40 °C to 80 °C.

According to an embodiment, there may be provided manufacturing an oil-dispersed double capsule in which one kind of oil is dispersed including
(a) manufacturing aqueous phase containing acrylate/C10-30alkylacrylatecrosspolymer, carbomer, agar, carrageenan, tromethamine, and purified water, and oil phase containing dimethicone, amodimethicone, dimethicone/vinyldimethicone crosspolymer and an encapsulating pigment; (b) manufacturing a first oil-dispersed double capsule base by mixing the oil phase with the aqueous phase obtained from (a) and heating to 80 °C to 100 °C; and (c) manufacturing an oil-dispersed double capsule by keeping the first oil-dispersed double capsule base obtained from (b) at the temperature of 40 °C to 80 °C and dropping to a dropping medium of 1 °C to 40 °C.

According to an embodiment, there may be provided manufacturing an oil-dispersed double capsule in which two kinds of oil are dispersed including
(a) manufacturing aqueous phase containing acrylate/C10-30alkylacrylatecrosspolymer, carbomer, agar, carrageenan, tromethamine, and purified water, first oil phase containing dimethicone, amodimethicone, dimethicone/vinyldimethicone crosspolymer and a first encapsulating pigment, and second oil phase containing dimethicone, amodimethicone, dimethicone/vinyldimethicone crosspolymer and a second encapsulating pigment; (b) manufacturing a second oil-dispersed double capsule base by sequentially mixing the first oil phase and the second oil phase to the aqueous phase obtained from (a) and heating to 80 °C to 100 °C; and (c) manufacturing an oil-dispersed double capsule by keeping the second oil-dispersed double capsule base obtained from (b) at the temperature of 40 °C to 80 °C and dropping to a dropping medium of 1 °C to 40 °C.

According to an embodiment, there may be provided manufacturing an oil-dispersed double capsule in which three kinds of oil are dispersed including
(a) manufacturing aqueous phase containing acrylate/C10-30alkylacrylatecrosspolymer, carbomer, agar, carrageenan, tromethamine and purified water, first oil phase containing dimethicone, amodimethicone, dimethicone/vinyldimethicone crosspolymer and a first encapsulating pigment, second oil phase containing dimethicone, amodimethicone, dimethicone/vinyldimethicone crosspolymer and a second encapsulating pigment, and third oil phase containing dimethicone, amodimethicone, dimethicone/vinyldimethicone crosspolymer and a third encapsulating pigment; (b) manufacturing a third oil-dispersed double capsule base by sequentially mixing the first oil phase, the second oil phase and the third oil phase to the aqueous phase obtained from (a) and heating to 80 °C to 100 °C; and (c) manufacturing an oil-dispersed double capsule by keeping the third oil-dispersed double capsule base obtained from (b) at 40 °C to 80 °C and dropping to a dropping medium of 1 °C to 40 °C.

According to another aspect of the invention, an oil-dispersed double capsule manufactured by the above manufacturing method is provided.

According to an embodiment, the oil-dispersed double capsule may have a size of 0.5 mm ― 50 mm.

### ADVANTAGEOUS EFFECTS OF INVENTION

According to the method for manufacturing an oil-dispersed double capsule of the invention, it is revealed that oil raw material can be secured within aqueous phase stably through utilization of proper thickener during capsule fabrication, complication of manufacturing installment according to cryogenic fabrication can be further simplified by solidifying slowly at 10 °C - 20 °C instead of cryogenic cooling method during bead formation process, and two kinds or more of oil-dispersed double capsule can be manufactured by using specific oils and pigments. Further, it is confirmed that there is no unique stickiness or smell of a surfactant when such an oil-dispersed double capsule is applied to the skin, and smooth crushing is available only with little pressure.

Accordingly, as the method for manufacturing an oil-dispersed double capsule of the present invention can stabilize poorly soluble active elements in the cosmetic manufacturing art and improve aesthetic sense through various color adjustment within the capsule, it may be commercialized as a skincare product or base makeup product and may be used as simple and efficient manufacturing technology for the mass production process.

### BRIEF DESCRIPTION OF DRAWINGS

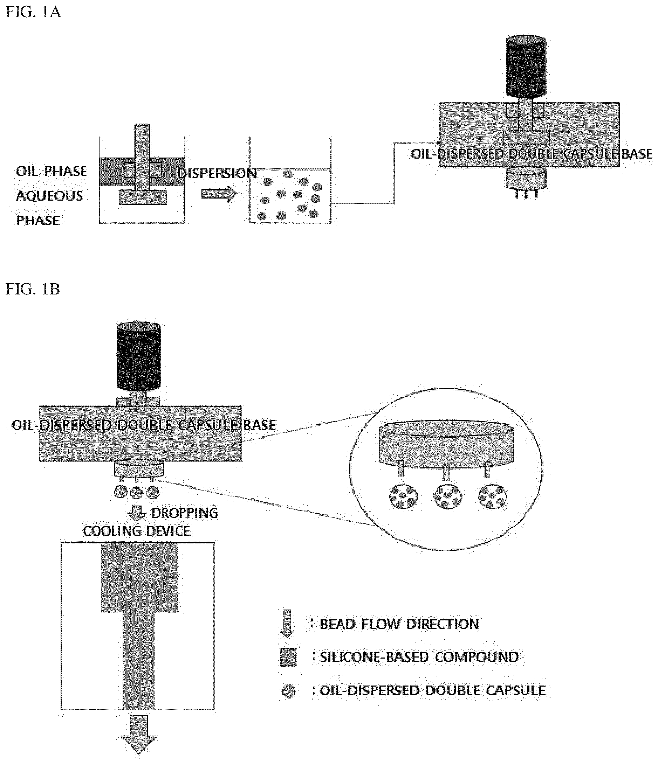

**FIG. 1** is a schematic diagram illustrating a manufacturing process of one kind of an oil-dispersed double capsule. FIG. 1A illustrates a manufacturing process of one kind of an oil-dispersed double capsule base, and FIG. 1B illustrates a manufacturing process of one kind of a beaded oil-dispersed double capsule.

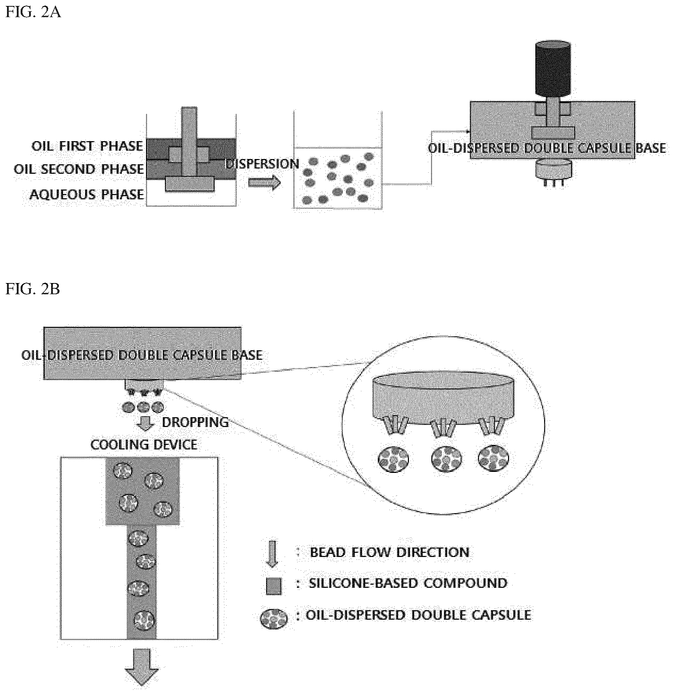

**FIG. 2** is a schematic diagram illustrating a manufacturing process of two kinds or more of an oil-dispersed double capsule. FIG. 2A illustrates a manufacturing process of two kinds of an oil-dispersed double capsule base, and FIG. 2B illustrates a manufacturing process of three kinds of a beaded oil-dispersed double capsule.

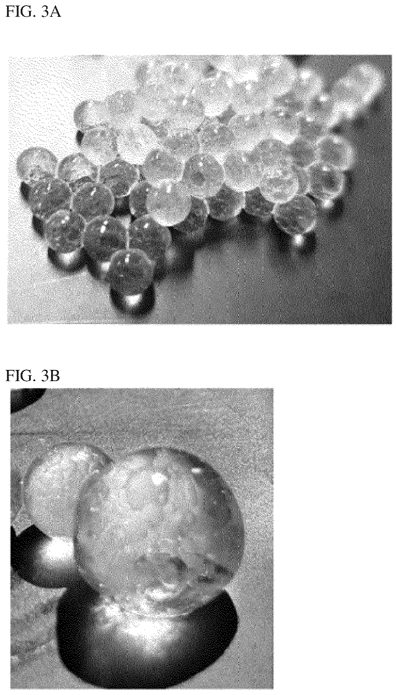

**FIG. 3** is a picture of one kind of an oil-dispersed double capsule. FIG. 3A illustrates a variety of one kind of oil-dispersed double capsules, and FIG. 3B magnifies one oil-dispersed double capsule having one kind (7 times).

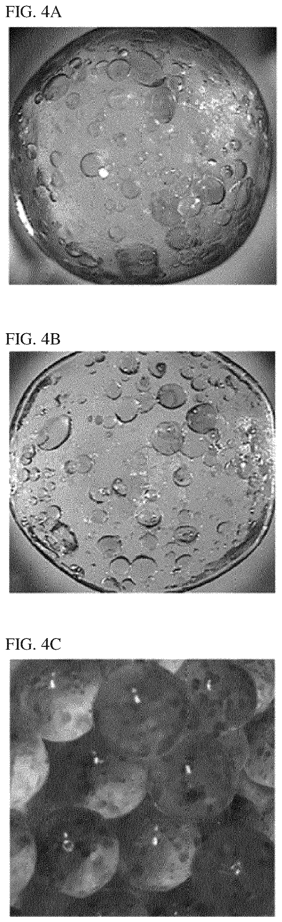

**FIG. 4** is a picture of two kinds of an oil-dispersed double capsule. FIG. 4A illustrates one oil-dispersed double capsule having two kinds, FIG. 4B illustrates a cross section of the oil-dispersed double capsule having two kinds, and FIG. 4C illustrates a variety of two kinds of oil-dispersed double capsules.

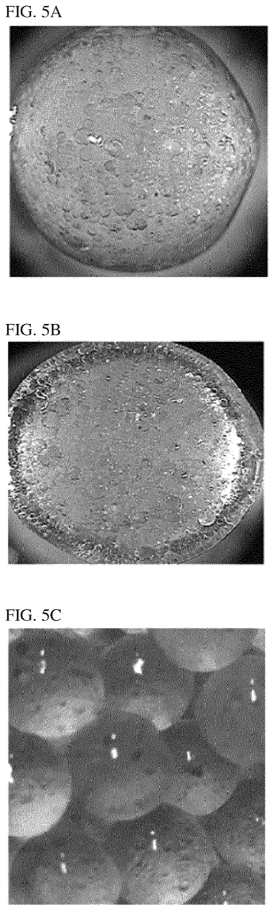

**FIG. 5** is a picture of three kinds of an oil-dispersed double capsule. FIG. 5A illustrates one oil-dispersed double capsule having three kinds, FIG. 5B illustrates a cross section of the oil-dispersed double capsule having three kinds, and FIG. 5C illustrates a variety of three kinds of oil-dispersed double capsules.

### BEST MODE FOR CARRYING OUT THE INVENTION

The present invention provides a method for manufacturing an oil-dispersed double capsule comprising (a) manufacturing aqueous phase including a thickener and a curing agent and oil phase including base oil and an encapsulating pigment; (b) manufacturing an oil-dispersed double capsule base by mixing the aqueous phase and the oil phase obtained from (a); and (c) manufacturing an oil-dispersed double capsule by dropping the oil-dispersed double capsule base obtained from (b) to a dropping medium.

According to an embodiment, one kind or more of oil may be dispersed in the oil-dispersed double capsule.

The manufacturing method of the present invention includes manufacturing the aqueous phase including a thickener and a curing agent, and the oil phase including base oil and an encapsulating pigment (i.e., step (a)). (a) is a step preparing aqueous phase and oil phase for manufacturing an oil-dispersed double capsule. Uniform mixture may be respectively prepared by adding elements corresponding to the aqueous phase and elements corresponding to the oil phase in each and stirring for a certain time.

At (a), a thickener to be used is a material used for increasing viscosity of the aqueous phase and may be used for clearly separating the oil phase as the oil phase is not easily emulsifying on the aqueous phase; preferably, one kind or more selected from a group consisting of acrylate/C10-30alkylacrylatecrosspolymer, carbomer, agar, carrageenan, guar gum, xanthan gum, alginic acid, pectin, starch, magnesium silicate, sodium polyacrylate starch and mixtures thereof may be used, and most preferably, acrylate/C10-30alkylacrylatecrosspolymer, carbomer, agar, carrageenan, and mixtures thereof may be used. Among them, agar, carrageenan, guar gum, xanthan gum and so on may be used as curing agent. Specifically, the aqueous phase may be used by properly adjusting a thickener and a curing agent so that viscosity becomes more than 10,000 cps at room temperature.

Further, the base oil to be used does not contain a surfactant and is an oil having polarity used to form oil phase on an upper layer of aqueous phase, and various elements that have been commonly used in the cosmetic industry may be used therein. Preferably, one kind or more selected from a group consisting of sunflower seed oil, macadamia seed oil, jojoba oil, drumstick seed oil, olive oil, grape seed oil, coconut oil, almond oil, camellia oil, avocado oil, caprlic/capric triglyceride, caprilic/capric glyceride, dimethicone, amodimethicone, phenyltrimethicone and dimethicone/vinyldimethicone crosspolymer may be used, and the base oil content may be included in 0.1 - 50 wt.%, preferably, 1 - 30 wt.%, and most preferably 3 - 15 wt.%.

The term, "encapsulating pigment", indicates pigment contained in a double capsule. Specifically, the pigment may use one kind or more selected from a group of inorganic pigments, e.g., ultramarine, red iron oxide, yellow iron oxide, and black iron oxide, and a group of oil soluble pigment, e.g., guaiazulene, Indian mulberry leaf extract, Indian mulberry flower extract, Indian mulberry gum extract, eclipta prostrata extract, ivy god fruit extract, eggplant extract, aloe vera extract, holy basil leaf extract, turmeric extract, and true coral extract. The double capsule of the present invention may include one type of pigment corresponding to one color only, or various types of pigments.

The encapsulating pigment may not be noticeable from an external surface of a capsule due to encapsulation but has advantage in which the pigment may be easily discharged externally from the micro capsule during application. The encapsulating pigment differs from a coating pigment used in a makeup product. In fact, while the coating pigment includes a chemical coating having a purpose of improving dispersion among compositions, encapsulating pigment includes one or more physical layer forming a coat where the layer is relatively uniform and isolated not to leak encapsulating pigment, such that each encapsulating pigment is properly individualized among composition, and this is different from a case of the coating pigment. According to the invention, aesthetic effect of appearance may be obtained through encapsulating pigment, and further, it is visually confirmed that one kind or more of oil element (active element) is secured stably within the capsule.

The manufacturing method of the present invention includes manufacturing an oil-dispersed double capsule base by mixing the aqueous phase and the oil phase obtained from (a) (i.e., step (b)). At (b), mixture (o/w) of mixing the aqueous phase and the oil phase is heated to 80 °C - 100 °C to lower viscosity.

At (b), a mixture in which the aqueous phase is added to the oil phase may be slowly stirred at a speed of 100 rpm - 150 rpm, and uniformly dispersed. Further, when two kinds or more of the oil phase are present, a first mixture (o/w) in which the aqueous phase is first mixed with one kind of the oil phase is sufficiently and uniformly stirred, and the remaining oil phase may be added and mixed. The above mixture sufficiently and uniformly dispersed may be processed by heating to 80 °C - 100 °C, preferably, to 85 °C - 95 °C. As viscosity may be lowered by performing the above heating process, and an oil-dispersed double capsule base having a flow is formed. It may be processed that the oil-dispersed double capsule base is passed through a nozzle after measuring viscosity and confirming proper viscosity.

Viscosity may be 300 cps to 1,500 cps after the heating process of the oil-dispersed double capsule base. When viscosity of the oil-dispersed double capsule base is 150 - 200 cps or less, a double capsule in which oil is uniformly dispersed may not be formed properly, and when viscosity is 2,000 cps or more, an oil-dispersed double capsule having a shape in which separation to each unit is not complete during a process of passing through the nozzle may be formed.

The manufacturing method of the present invention includes manufacturing an oil-dispersed double capsule by dropping the oil-dispersed double capsule base obtained from (b) to a dropping medium (i.e., step (c)). (C) is a step in which the oil-dispersed double capsule is obtained from the oil-dispersed double capsule base. At (c), the double capsule bead passing through the nozzle from the oil-dispersed double capsule base kept at the temperature of 40 °C - 80 °C may be processed to be dropped to a cooling device having a dropping medium in a range of 1 °C - 40 °C.

The oil-dispersed double capsule base may be kept at a temperature in the range of 40 °C - 80 °C, preferably, in the range of 50 °C - 75 °C, and most preferably, in the range of 60 °C - 70 °C.

The dropping medium may be set at a temperature in the range of 1 °C - 40 °C, and preferably, in the range of 10 °C - 20 °C. The dropping medium may use oil which is liquid and has fluidity such as silicone oil and mineral oil. Silicone oil and mineral oil may be preferably used because there are little viscosity changes according to changes in temperature. As an example of silicone oil, there may be provided dimethyl silicone oil, methylphenyl silicone oil, methylhydrogen silicone oil, methylhydroxy silicone oil, fluoro silicone oil, polyoxyether copolymer, alkyl-modified silicone oil, higher fatty acid-modified silicone oil, amino-modified silicone oil, and epoxy-modified silicone oil. Mineral oil may be called as fluid oil or mineral oil, and mean by-product generated from a process of refining crude into oil, and main elements are alkane and paraffin.

According to an embodiment, manufacturing an oil-dispersed double capsule in which one kind of oil is dispersed may include (a) manufacturing aqueous phase containing acrylate/C10-30alkylacrylatecrosspolymer, carbomer, agar, carrageenan, tromethamine, and purified water, first oil phase containing dimethicone, amodimethicone, dimethicone/vinyldimethicone crosspolymer, and a first encapsulating pigment, and second oil phase containing dimethicone, amodimethicone, dimethicone/vinyldimethicone crosspolymer, and a second encapsulating pigment; (b) manufacturing a second oil-dispersed double capsule base by sequentially mixing the first oil phase and the second oil phase in the aqueous phase obtained from (a) and heating to 80 °C to 100 °C; and (c) manufacturing an oil-dispersed double capsule by dropping the second oil-dispersed double capsule base obtained from (b) to a dropping medium of 1 °C - 40 °C while keeping the oil-dispersed double capsule base at the temperature of 40 °C - 80 °C.

According to an embodiment, manufacturing an oil-dispersed double capsule in which two kinds of oil are dispersed may include (a) manufacturing aqueous phase containing acrylate/C10-30alkylacrylratecrosspolymer, carbomer, agar, carrageenan, tromethamine and purified water, first oil phase containing dimethicone, amodimethicone, dimethicone/vinyldimethicone crosspolymer and a first encapsulating pigment, second oil phase containing dimethicone, amodimethicone, dimethicone/vinyldimethicone crosspolymer and a second encapsulating pigment, and third oil phase containing dimethicone, amodimethicone, dimethicone/vinyldimethicone crosspolymer, and a third encapsulating pigment; (b) manufacturing a third oil-dispersed double capsule base by sequentially mixing the first oil phase, the second oil phase and the third oil phase in the aqueous phase obtained from (a) and heating to 80 °C - 100 °C; and (c) manufacturing an oil-dispersed double capsule by dropping the third oil-dispersed double capsule base obtained from (b) to a dropping medium of 1 °C - 40 °C while keeping the third oil-dispersed double capsule base at the temperature of 40 °C - 80 °C.

According to an embodiment, there may be provided manufacturing an oil-dispersed double capsule in which three kinds of oil are dispersed including
(a) manufacturing aqueous phase containing acrylate/C10-30alkylacrylatecrosspolymer, carbomer, agar, carrageenan, tromethamine and purified water, first oil phase containing dimethicone, amodimethicone, dimethicone/vinyldimethicone crosspolymer and a first encapsulating pigment, second oil phase containing dimethicone, amodimethicone, dimethicone/vinyldimethicone crosspolymer and a second encapsulating pigment, and third oil phase containing dimethicone, amodimethicone, dimethicone/vinyldimethicone crosspolymer and a third encapsulating pigment; (b) manufacturing a third oil-dispersed double capsule base by sequentially mixing the first oil phase, the second oil phase and the third oil phase to the aqueous phase obtained from (a) and heating to 80 °C to 100 °C; and (c) manufacturing an oil-dispersed double capsule by keeping the third oil-dispersed double capsule base obtained from (b) at the temperature of 40 °C to 80 °C and dropping to a dropping medium of 1 °C to 40 °C.

Further, the present invention provides an oil-dispersed double capsule manufactured by the above manufacturing method. The oil-dispersed double capsule may have a size (diameter or longest distance) of 0.5 mm - 50 mm, preferably, 1 mm - 10 mm, and most preferably, 5 mm - 8 mm. With respect to droplets of the oil-dispersed double capsule base, a desired size and/or shape may be appropriately selected by properly adjusting diameter of the nozzle and pressure of a spray.

The double capsule has a semitransparent ivory white color as a whole and one kind or more of colors may be applied according to ratio of each encapsulating pigment through diversification of oil color within the capsule, and therefore, aesthetic sense is confirmed to be improved (FIGS. 3 to 5). Further, in the sensory evaluation related with the feeling of use of an oil-dispersed double capsule, each having oil content of 3 wt.% - 15 wt.%, preference of texture increases as oil content increases, and the feeling of use is confirmed to be enhanced.

Because the above double capsule is encapsulated with aqueous carrier having no influence on general cosmetic base elements, oil elements containing a variety of active elements may be stably secured within the capsule in manufacturing cosmetic products. Further, cosmetic composition containing an oil-dispersed double capsule may be simply adjusted from the oil phase in the fabrication process with respect to color, size, and active element content of the interior of the capsule. Further, formulation of cosmetics produced therefrom has advantage in which visual effect is improved.

Hereinbelow, the present invention will be described with more details in embodiments. However, following embodiments are exemplified and illustrative for the present invention, and the scope of the present invention is not limited thereto.

### Embodiment 1: Manufacturing of one kind of oil-dispersed double capsule

Elements of an oil-dispersed double capsule using one kind of color are described in a following Table 1.

**[Table 1]**

| Phase | Ingredients | Mixure ratio (wt.%) | Function |
|---|---|---|---|
| Aqueous phase | purified water | 86.08 | solvent |
| | 1,2-hexanediol | 1.60 | preservative |
| | ethylhexylglycerine | 0.08 | preservative |
| | acrylate/C10-30alkylacrylatecrosspolymer | 0.10 | thickener |
| | carbomer | 0.12 | |
| | tromethamine | 0.10 | counteractive |
| | agar | 0.70 | thickener/curing agent |
| | carrageenan | 0.35 | |
| Oil phase | dimethicone | 0.96 | oil |
| | amodimethicone | 0.24 | modified silicone (oil) |
| | dimethicone/vinyldimethicone crosspolymer | 9.60 | high-viscosity silicone (oil) |
| | ultramarine pigment | 0.08 | encapsulating pigment |

Elements of the above Table 1 are classified as follows and fabrication process is performed (FIGS. 1A and 1B).
(1) Aqueous phase dissolving tank: Purified water, 1,2-hexanediol, ethylhexylglycerine, acrylate/C10-30alkylacrylatecrosspolymer, carbomer, tromethamine, agar, carrageenan
(2) Oil phase dissolving tank: Dimethicone, amodimethicone, dimethicone/vinyldimethicone crosspolymer, ultramarine pigment

First, raw material is weighed in amount described in Table 1 with a micro scale, and elements corresponding to the aqueous phase are added and stirred at 500 rpm for 10 minutes to be dispersed uniformly. Next, raw material corresponding to the oil phase is stirred at 300 rpm for 5 minutes to be dispersed uniformly.

An oil-dispersed double capsule base having a flow is manufactured by adding the oil phase into the uniformly dispersed aqueous phase, which is slowly stirred at 100 rpm - 150 rpm for 5 - 7 minutes and uniformly dispersed, then, heating to 90 °C, stirring at a speed of 100 rpm for 10 minutes, and lowering the temperature to 67 °C - 70 °C.

A capsule is obtained having a cross section in oil dispersion liquid in contact by passing the manufactured oil-dispersed double capsule base through a nozzle. After the obtained double capsule is dropped to a dropping medium kept at 10 °C - 20 °C (silicone-based compound) in each drop, it is cooled and becomes solid during circulation, and discharged in solid state, and a double capsule evenly dispersed with oil liquid in marine color is obtained. The oil-dispersed double capsule obtained in a round shape has a size of about 4.5 mm - 7 mm and weight composition of Table 1. Pictures of the obtained product are provided in FIGS. 3A and 3B.

Further, in the sensory evaluation related with the feeling of use of the oil-dispersed double capsule having each of oil content of 3 wt.% - 15 wt.%, as oil content increases, the feeling of use is confirmed to be enhanced.

### Embodiment 2: manufacturing of two kinds of oil-dispersed double capsule

Elements of an oil-dispersed double capsule having two kinds of color are described in a following Table 2.

**[Table 2]**

| Phase | Ingredients | Mixure ratio (wt.%) | Function |
|---|---|---|---|
| Aqueous phase | purified water | 92.26 | solvent |
| | 1,2-hexanediol | 1.60 | preservative |
| | ethylhexylglycerine | 0.08 | preservative |
| | acrylate/C10-30alkylacrylatecrosspolymer | 0.11 | thickener |
| | carbomer | 0.13 | |
| | tromethamine | 0.11 | counteractive |
| | agar | 0.70 | thickener/curing agent |
| | carrageenan | 0.35 | |
| Oil phase 1 | dimethicone | 0.02 | oil |
| | amodimethicone | 0.05 | modified silicone (oil) |
| | dimethicone/vinyldimethicone crosspolymer | 2.20 | high-viscosity silicone (oil) |
| | ultramarine pigment | 0.03 | encapsulating pigment |
| Oil phase 2 | dimethicone | 0.02 | oil |
| | amodimethicone | 0.05 | modified silicone (oil) |
| | dimethicone/vinyldimethicone crosspolymer | 2.20 | high-viscosity silicone (oil) |
| | red iron oxide | 0.10 | encapsulating pigment |

Fabrication process identical to Embodiment 1 is performed by using constituent elements of the above Table 2 (FIG. 2A).

With the method identical to Embodiment 1, an aqueous phase dissolving tank, an oil phase dissolving tank 1 and an oil phase dissolving tank 2 are prepared.

Further, an oil-dispersed double capsule base having a flow is manufactured by sequentially adding the oil phase 1 and the oil phase 2 into the uniformly dispersed aqueous phase and using the method identical to Embodiment 1.

Pictures of the oil-dispersed double capsule in a round shape having two colors obtained from the manufactured oil-dispersed double capsule base are shown in FIGS. 4A, 4B, and 4C.

### Embodiment 3: manufacturing of three kinds of oil-dispersed double capsule

Elements of an oil-dispersed double capsule using three kinds of color are described in a following Table 3.

**[Table 3]**

| Phase | Ingredients | Mixure ratio (wt.%) | Function |
|---|---|---|---|
| Aqueous phase | purified water | 92.15 | solvent |
| | 1,2-hexanediol | 1.60 | preservative |
| | ethylhexylglycerine | 0.08 | preservative |
| | acrylate/C10-30alkylacrylatecrosspolymer | 0.11 | thickener |
| | carbomer | 0.13 | |
| | tromethamine | 0.11 | counteractive |
| | agar | 0.70 | thickener/curing agent |
| | carrageenan | 0.35 | |
| Oil phase 1 | dimethicone | 0.01 | oil |
| | amodimethicone | 0.03 | modified silicone (oil) |
| | dimethicone/vinyldimethicone crosspolymer | 1.50 | high-viscosity silicone (oil) |
| | ultramarine pigment | 0.05 | encapsulating pigment |
| Oil phase 2 | dimethicone | 0.01 | oil |
| | amodimethicone | 0.03 | modified silicone (oil) |
| | dimethicone/vinyldimethicone crosspolymer | 1.50 | high-viscosity silicone (oil) |
| | red iron oxide | 0.02 | encapsulating pigment |
| Oil phase 3 | dimethicone | 0.01 | oil |
| | amodimethicone | 0.03 | modified silicone (oil) |
| | dimethicone/vinyldimethicone crosspolymer | 1.50 | high-viscosity silicone (oil) |
| | yellow iron oxide | 0.09 | encapsulating pigment |

Fabrication process identical to Embodiment 1 is performed by using constituent elements of the above Table 3 (FIG. 2B).

With the method identical to Embodiment 1, an aqueous phase dissolving tank, an oil phase dissolving tank 1, an oil phase dissolving tank 2 and an oil phase dissolving tank 3 are prepared.

Further, an oil-dispersed double capsule base having a flow is manufactured by sequentially adding the oil phase 1, the oil phase 2, and the oil phase 3 in the uniformly dispersed aqueous phase and using the method identical to Embodiment 1.

Pictures of the oil-dispersed double capsule in a round shape having three colors obtained from the manufactured oil-dispersed double capsule base are shown in FIGS. 5A, 5B, and 5C.

### Comparative examples 1 to 2: manufacturing of one kind of an oil-dispersed double capsule having different thickener conditions

Content per component of a thickener in Embodiment 1 and one kind of an oil-dispersed double capsule using one color having different thickener conditions is described in a following Table 4. Comparative examples 1 to 2 show an oil-dispersed double capsule not containing acrylate/C10-30alkylacrylatecrosspolymer or carbomer among thickener elements. Differing from Embodiment 1, oil is not uniformly dispersed, stability of an oil film within the water carrier is lowered, and aesthetic sense is significantly degraded in Comparative examples 1 to 2. Accordingly, in manufacturing an oil-dispersed double capsule, it is confirmed that viscosity more than 10,000 cps in the aqueous phase at room temperature before heating is critical.

**[Table 4]**

| Phase | Ingredients | Em. 1 | Com. 1 | Com. 2 |
|---|---|---|---|---|
| Thickener | acrylate/C10-30alkylacrylatecrosspolymer | 0.10 | - | 0.10 |
| | carbo mer | 0.12 | 0.12 | - |
| Thickener and curing agent | agar | 0.70 | 0.70 | 0.70 |
| | carrageenan | 0.35 | 0.35 | 0.35 |

## Claims

1. A method for manufacturing an oil-dispersed double capsule, comprising:
(a) manufacturing aqueous phase containing a thickener and a curing agent and oil phase containing base oil and an encapsulating pigment;
(b) manufacturing an oil-dispersed double capsule base by mixing the aqueous phase and the oil phase obtained from (a); and
(c) manufacturing an oil-dispersed double capsule by dropping the oil-dispersed double capsule base obtained from (b) to a dropping medium.

2. The method of claim 1, wherein one kind or more of oil is dispersed in the oil-dispersed double capsule.

3. The method of claim 1, wherein the thickener of (a) is one kind or more selected from a group consisting of acrylate/C10-30alkylacrylatecrosspolymer, carbomer, agar, carrageenan, guar gum, xanthan gum, alginic acid, pectin, starch, magnesium silicate, and sodium polyacrylate starch; and the base oil is one kind or more selected from a group consisting of sunflower seed oil, macadamia seed oil, jojoba oil, drumstick seed oil, olive oil, grape seed oil, coconut oil, almond oil, camellia oil, avocado oil, caprlic/caprictriglyceride, caprlic/capricglyceride, dimethicone, amodimethicone, penyltrimethicone and dimethicone/vinyldimethicone crosspolymer.

4. The method of claim 1, wherein the base oil of (a) comprises 0.1 to 50 wt.%.

5. The method of claim 1, wherein the oil-dispersed double capsule base of (c) is kept at the temperature of 40 °C to 80 °C.

6. The method of claim 1, wherein manufacturing an oil-dispersed double capsule in which one kind of oil is dispersed comprises
(a) manufacturing aqueous phase containing acrylate/C10-30alkylacrylatecrosspolymer, carbomer, agar, carrageenan, tromethamine, and purified water, and oil phase containing dimethicone, amodimethicone, dimethicone/vinyldimethicone crosspolymer and an encapsulating pigment;
(b) manufacturing a first oil-dispersed double capsule base by mixing the oil phase with the aqueous phase obtained from (a) and heating to 80 °C to 100 °C; and
(c) manufacturing an oil-dispersed double capsule by keeping the first oil-dispersed double capsule base obtained from (b) at the temperature of 40 °C to 80 °C and dropping to a dropping medium of 1 °C to 40 °C.

7. The method of claim 1, wherein manufacturing an oil-dispersed double capsule in which two kinds of oil are dispersed comprises
(a) manufacturing aqueous phase containing acrylate/C10-30alkylacrylatecrosspolymer, carbomer, agar, carrageenan, tromethamine, and purified water, first oil phase containing dimethicone, amodimethicone, dimethicone/vinyldimethicone crosspolymer and a first encapsulating pigment, and second oil phase containing dimethicone, amodimethicone, dimethicone/vinyldimethicone crosspolymer and a second encapsulating pigment;
(b) manufacturing a second oil-dispersed double capsule base by sequentially mixing the first oil phase and the second oil phase to the aqueous phase obtained from (a) and heating to 80 °C to 100 °C; and
(c) manufacturing an oil-dispersed double capsule by keeping the second oil-dispersed double capsule base obtained from (b) at the temperature of 40 °C to 80 °C and dropping to a dropping medium of 1 °C to 40 °C.

8. The method of claim 1, wherein manufacturing an oil-dispersed double capsule in which three kinds of oil are dispersed comprises
(a) manufacturing aqueous phase containing acrylate/C10-30alkylacrylatecrosspolymer, carbomer, agar, carrageenan, tromethamine and purified water, first oil phase containing dimethicone, amodimethicone, dimethicone/vinyldimethicone crosspolymer and a first encapsulating pigment, second oil phase containing dimethicone, amodimethicone, dimethicone/vinyldimethicone crosspolymer and a second encapsulating pigment, and third oil phase containing dimethicone, amodimethicone, dimethicone/vinyldimethicone crosspolymer and a third encapsulating pigment;
(b) manufacturing a third oil-dispersed double capsule base by sequentially mixing the first oil phase, the second oil phase and the third oil phase to the aqueous phase obtained from (a) and heating to 80 °C to 100 °C; and
(c) manufacturing an oil-dispersed double capsule by keeping the third oil-dispersed double capsule base obtained from (b) at 40 °C to 80 °C and dropping to a dropping medium of 1 °C to 40 °C.

9. An oil-dispersed double capsule manufactured by a manufacturing method of any one of claims 1 to 8.

10. The method of claim 9, wherein the oil-dispersed double capsule has a size of 0.5 mm - 50 mm.
